# EUROPÄISCHE PATENTSCHRIFT

(11) **EP 0 510 424 B1**
(45) Veröffentlichungstag und Bekanntmachung des Hinweises auf die Patenterteilung: **12.07.1995**
(21) Anmeldenummer: 92106021.6
(22) Anmeldetag: 07.04.1992
(51) Int. Cl.: A61B 6/00

(54) **Röntgendiagnostikgerät für Mammografieuntersuchungen**
X-ray diagnosis apparatus for mammography
Appareil radiodiagnostique pour la mammographie

(30) Priorität: 23.04.1991 SE 9101216
(43) Veröffentlichungstag der Anmeldung: 28.10.1992
(73) Patentinhaber: Siemens-Elema AB, 171 95 Solna 1 (SE); SIEMENS AKTIENGESELLSCHAFT, 80333 München (DE)
(72) Erfinder: Thunberg, Stefan, S-116 58 Stockholm (SE); Johansson, Per, S-196 31 Kungsängen (SE)

(56) Entgegenhaltungen:
- DE-A- 3 319 622
- SE-B- 466 333
- US-A- 4 394 771
- US-A- 4 879 736

## Beschreibung

Die Erfindung betrifft ein Röntgendiagnostikgerät für Mammografieuntersuchungen mit einer Röntgenröhre, die über eine horizontale Welle um ein in einem Stativ angeordnetes Lager drehbar ist, und mit mindestens zwei Kassettenhalterungen, die wahlweise in eine Aufnahmelage gebracht werden können, wobei die Kassettenhalterungen um die horizontale Welle derart schwenkbar sind, daß sich, wenn eine Kassettenhalterung in die Aufnahmelage gebracht ist, die andere Kassettenhalterung in einer Parklage befindet.

Ein Röntgendiagnostikgerät dieser Art ist durch die DE-OS 33 19 622 bekannt. Die Kassettenhalterungen für Röntgenfilmkassetten verschiedener Größen sind an einer Halterung befestigt, die um die horizontale Welle, die etwa zwischen der Röntgenröhre und der Bildebene angebracht ist, schwenkbar befestigt ist. Hierdurch kann die Halterung derart ausgebildet werden, daß sich die Kassettenhalterungen in ihren Parklagen sich genau oberhalb der Röntgenröhre befinden. Die Halterung mit den Kassettenhalterungen, die eine U-Form bildet, ist auf diese Weise verhältnismäßig groß und kann daher bei einem Positionswechsel einer Kassettenhalterung von einer Aufnahmelage in eine Parklage etwas schwer zu manövrieren sein und gleichzeitig einen unförmigen Eindruck erwecken.

Der Erfindung liegt die Aufgabe zugrunde, ein Röntgendiagnosikgerät der eingangs genannten Art zu schaffen, bei dem die Röntgenröhre so gedreht werden kann, daß die horizontale Welle in einer gewünschten Lage ihre vertikale Position zwischen den Aufnahmen beibehalten kann, gleichzeitig damit, daß der Patient stehen bleiben kann. Ferner ist eine Positionswechselanordnung für die Kassettenhalterungen, die verhältnismäßig klein und leicht zu handhaben ist, angestrebt.

Diese Aufgabe ist erfindungsgemäß dadurch gelöst, daß die Drehachse der horizontalen Welle etwa achsfluchtend mit der Mitte des Untersuchungsobjekts angeordnet ist und daß die Kassettenhalterungen in einer Halterung, die an der horizontalen Welle drehbar angebracht ist, derart befestigt sind, daß sich, wenn die eine Kassettenhalterung in die Aufnahmelage gebracht ist, die andere Kassettenhalterung vom Untersuchungsobjekt her gesehen, in einer Parklage hinter der Röntgenröhre befindet. Dabei erfolgt die Drehung der Halterung um die Welle derart, daß die Kassettenhalterungen jeweils etwa denselben Abstand zur Drehachse der horizontalen Welle aufweisen, wenn sie sich in der Aufnahmelage befinden. Ein Röntgendiagnostikgerät dieser Art mit einer Welle für die Drehung der Röntgenröhre, deren Drehzentrum etwa mit der Mitte des Untersuchungsobjekts zusammenfällt, ist in der SE-Anmeldung 9100362-4 dargestellt und beschrieben. Die Positionswechselanordnung für die Kassettenhalterungen gemäß der Erfindung ist insbesondere für ein Röntgendiagnostikgerät dieser Art geeignet. Dadurch, daß sich die Kassettenhalterungen in der Parklage im Raum zwischen der Röntgenröhre und dem Stativ befinden, sind die Kassettenhalterungen in dieser Position den Bedienungspersonen nicht im Wege. Außerdem bekommt der Betrachter den Eindruck einer eleganten Konstruktion. Ferner kann die Positionswechselanordnung durch diesen Aufbau klein gehalten werden und ist dadurch leicht zu handhaben.

In einer vorteilhaften Ausbildung der Erfindung wird vorgeschlagen, daß die Halterung an der horizontalen Welle in einem Winkel von etwa 45° angebracht ist. Dadurch können die Kassettenhalterungen, im Profil betrachtet, an der Halterung derart angebracht werden, daß sie einen gegenseitigen Winkel von 90° aufweisen.

Eine vorteilhafte Weiterbildung der Erfindung ist gegeben, indem die Halterung drei Kassettenhalterungen in einem Winkelabstand von jeweils 120° trägt. Die dritte Kassettenhalterung kann z.B. eine digitale Platte sein, die bei Screening verwendet werden kann. Durch den erwähnten Winkelabstand zwischen den Kassettenhalterungen sind die weiteren geparkten Kassettenhalterungen nicht im Wege, wenn die dritte Kassettenhalterung in eine Aufnahmelage gebracht worden ist.

Im Hinblick auf eine weitere Ausgestaltung der Erfindung wird vorgeschlagen, daß an der horizontalen Welle ein Lagergehäuse angebracht ist, an dem eine Lagerscheibe in einem Winkel von etwa 45° zur genannten Welle befestigt ist, wobei die Halterung an der Lagerscheibe angebracht ist. Hierdurch wird ein sehr einfacher Aufbau der Befestigung der Halterung an der Welle erhalten.

In einer konstruktiv einfachen Ausgestaltung der Erfindung empfiehlt es sich, daß das Lagergehäuse mit mindestens einem Flansch versehen ist, an dem die Lagerscheibe befestigt ist. Durch diesen Aufbau ist eine einfache und stabile Verbindung zwischen dem Lagergehäuse und der Lagerscheibe gegeben.

Im Hinblick auf eine vorteilhafte Weiterentwicklung der Erfindung wird vorgeschlagen, daß die Halterung mittels eines Ringes und eines Deckels für den Ring um die Lagerscheibe drehbar angebracht ist, wobei zwischen der Lagerscheibe und dem Ring und der Lagerscheibe und dem Deckel Gleitlager angeordnet sind. Hierdurch ist sichergestellt, daß die Halterung leicht um die Lagerscheibe gedreht werden kann.

In einer weiteren, kontruktiv einfachen Ausführung der Erfindung wird vorgeschlagen, daß die horizontale Welle, die zusammen mit der Röntgenröhre, dem Lagergehäuse und der Halterung für die Kassettenhalterungen um das Lager im Stativ drehbar ist, mittels eines Verschlußteils, das mit dem Verschlußkragen in einer der Arretierlagen verschließbar ist, in bestimmten Lagen arretierbar ist. Auf diese Weise kann die Röntgenröhre leicht in eine gewünschte Winkellage eingestellt werden.

Weitere Vorteile und Einzelheiten ergeben sich aus den Unteransprüchen.

Die Erfindung ist nachfolgend anhand eines in den Zeichnungen dargestellten Ausführungsbeispiels näher erläutert.
Es zeigen:
- FIG 1: eine Seitenansicht eines Röntgendiagnostikgeräts mit einer Wechseleinrichtung für Kassettenhalterungen nach der Erfindung und
- FIG 2: eine Seitenansicht einer Wechseleinrichtung nach FIG 1 im Schnitt.

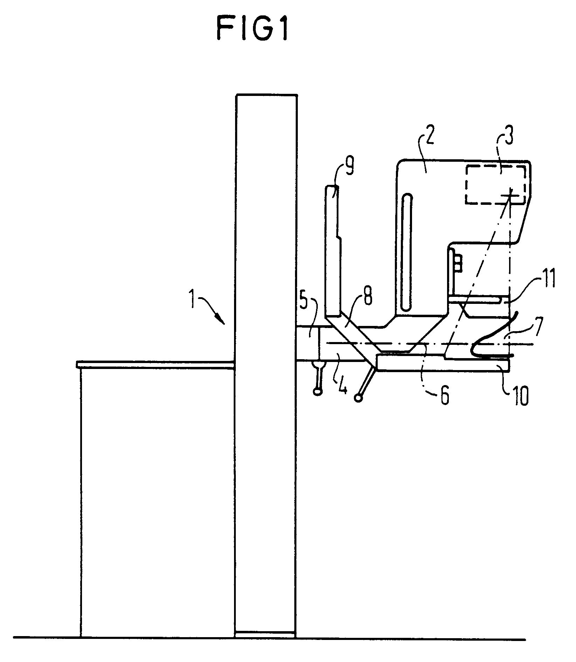

In der FIG 1 ist ein Röntgendiagnostikgerät für Mammografieuntersuchungen mit einem Stativ 1 gezeigt, das ein Gehäuse 2 beinhaltet, welches eine Röntgenröhre 3 trägt. Das Gehäuse 2 ist über eine horizontale Welle 4 um ein im Stativ 1 angeordnetes Lager 5, das in der vertikalen Richtung verschiebbar ist, drehbar angebracht. Das Drehzentrum 6 der horizontalen Welle 4 ist etwa achsfluchtend mit der Mitte des Untersuchungsobjekts 7 angeordnet. An der horizontalen Welle 4 ist eine Halterung 8 für zwei Kassettenhalterungen 9,10 für Röntgenfilmkassetten verschiedener Größen drehbar angebracht. Die Kassettenhalterungen 9,10 sind an der Halterung 8 in einem Winkelabstand von 180° derart angebracht, daß sich, wenn die eine Kassettenhalterung 10 in eine Aufnahmelage gebracht ist, die andere Kassettenhalterung 9, vom Untersuchungsobjekt 7 her gesehen, in einer Parklage hinter dem Gehäuse 2 für die Röntgenröhre 3 befindet. Wenn ein Untersuchungsobjekt 7 einer bestimmten Größe aufgenommen werden soll, wird mit Hilfe von später beschriebenen Mitteln die für diese Größe am meisten geeignete Kassettenhalterung von einer Parklage in eine Aufnahmelage gedreht. Die Drehung der Halterung 8 um die Welle 4 erfolgt dabei derart, daß die Kassettenhalterungen 9,10 jeweils etwa denselben Abstand zur Drehachse der horizontalen Welle 4 aufweisen, wenn sie sich in der Aufnahmelage befinden. Wie in der Figur gezeigt ist, dienen die Kassettenhalterungen 9,10 als Objekttisch, wenn diese eine Aufnahmeposition eingenommen haben. Am Gehäuse 2 ist eine Kompressionsplatte 11 in der Höhe verschiebbar angeordnet, die vor einer Aufnahme das Untersuchungsobjekt 7 gegen die Kassettenhalterung 9,10 drückt. Das Gehäuse 2 mit der Röntgenröhre 3 kann mittels der horizontalen Welle 4 um das Lager 5 am Stativ 1 in eine weitere gewünschte Aufnahmelage gedreht werden. Bei einer derartigen Drehung wird auch die Halterung 8 mit den Kassettenhalterungen 9,10 gedreht. Die Kassettenhalterungen 9,10 können auch in einer solchen Schräglage von einer Parklage in eine Aufnahmelage gewechselt werden.

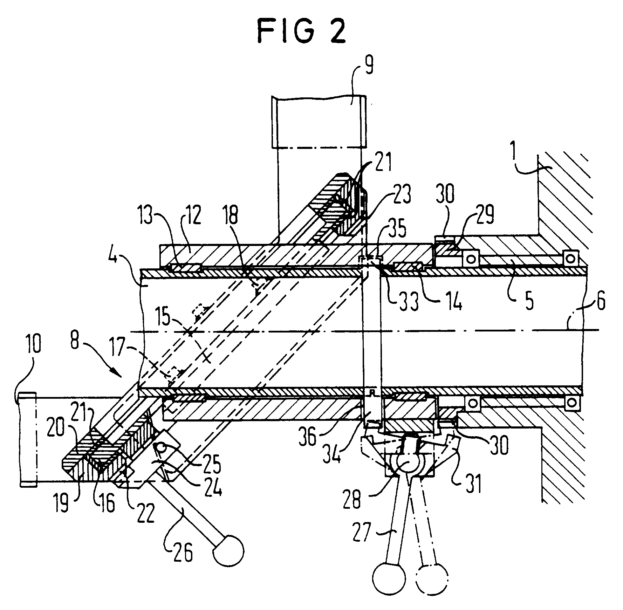

In der FIG 2 ist näher gezeigt, wie die horizontale Welle 4 um das Lager 5 am Stativ 1 drehbar angebracht ist. Die Welle 4 ist mit einem Lagergehäuse 12 mit Gleitlagren 13,14 versehen. Auf diese Weise kann die Welle 4 im Verhältnis zum Lagergehäuse 12 gedreht werden. Das Lagergehäuse 12 ist seinerseits mit Flanschen 15 versehen, die mit einem Winkel von etwa 45° zur horizontalen Welle 4 angebracht sind und an welchen eine Lagerscheibe 16 mittels Schrauben 17,18 befestigt ist. In der FIG 2 wird lediglich einer der Flansche 15 gezeigt. Die Halterung 8 ist mit einem Ring 19 und einem Deckel 20 für den Ring 19 versehen, die die Lagerscheibe 16, um die die Halterung 8 drehbar ist, umgeben. Zwischen der Lagerscheibe 16 und dem Ring 19 und der Lagerscheibe 16 und dem Deckel 20 sind Gleitlager 21 vorgesehen, die die Drehung der Halterung 8 um die Lagerscheibe 16 erleichtern. Die Kassettenhalterungen 9,10 sind, wie bereits erwähnt, an der Halterung 8 in einem Winkelabstand von 180° angebracht. Der Deckel 20 ist mit einer Arretiernut 22,23 zur Arretierung der Kassettenhalterungen 9,10 in ihren respektiven Aufnahmelagen versehen. Wenn eine der Kassenhalterungen 9,10 in eine gewünschte Aufnahmelage gebracht ist, steht die Arretiernut, z.B. 22, genau vor einem Verschlußteil 24, das durch eine Welle 25 am Lagergehäuse 12 drehbar angebracht ist und das mit Hilfe eines Hebels 26 in die Arretiernut 22 heruntergesenkt werden kann, wobei die Kassettenhalterung 10 in ihrer Lage arretiert wird. Bei einem Wechseln der Kassettenhalterungen 9,10 wird das Verschlußteil 24 mittels des Hebels 26 aus der Arretiernut 22 herausgeführt, wobei die Halterung 8 mit Hilfe des Ringes 19 und des Deckels 20, 180° um die Lagerscheibe 16 gedreht wird, bis die Arretiernut 23 der Kassettenhalterung 9 genau vor dem Verschlußteil 24 steht, das wie beschrieben die Kassettenhalterung 9 in einer Aufnahmelage arretiert.

In der FIG 2 wird auch gezeigt, daß ein weiterer Hebel 27 mit einem Verschlußteil 31 um eine Welle 28 im Lagergehäuse 12 drehbar befestigt ist. Im Stativ 1 und im Anschluß zum Lager 5 ist ein Verschlußkragen 29 befestigt, der die Welle 4 umschließt. Der Verschlußkragen 29 ist mit Arretiernuten 30 versehen, die an der Peripherieoberfläche des Verschlußkragens 29 z.B. in einem Winkelabstand von jeweils 15° gleich verteilt angebracht sind. Wenn der Hebel 27 in eine Lage gebracht worden ist, wie die strichpunktierte Version des Hebels 27 zeigt, d.h. wenn das Verschlußteil 31 in eine Arretiernut 30 einrastet, ist die Welle 4 mit dem Gehäuse 2 und die Röntgenröhre 3 in dieser Lage arretiert. Wenn das Gehäuse 2 mit der Röntgenröhre 3 zusammen mit der Welle 4 in eine gewünschte schräge Aufnahmelage gedreht werden soll, wird das Verschlußteil 31 mit Hilfe des Hebels 27 aus der Arretiernut 30 herausgeführt, wonach die Welle 4 mit dem Gehäuse 2 und die Röntgenröhre 3 in die gewünschte Lage gedreht werden kann. In dieser Lage kann die Welle 4, wie beschrieben, mittels des Verschlußteils 31 wieder arretiert werden. Bei dieser Drehung der Welle 4 wird auch das Lagergehäuse 12 mit der Halterung 8 gedreht.

Wie bereits beschrieben kann auch die Welle 4 gegenüber dem Lagergehäuse 12 gedreht werden. Dies erfolgt im Zusammenhang mit stereotaktischen Aufnahmen. Bei solchen Aufnahmen wird die Röntgenröhre 3 im Verhältnis zur Kassettenhalterung 9,10 in einem Winkel von etwa 3,5° in beiden Richtungen im Vergleich zu einer senkrechten Aufnahme gedreht. Bei derartigen Aufnahmen wird das Lagergehäuse 12 in beschriebener Weise mittels des Verschlußkragens 29 arretiert. Die Enden 33,34 einer Welle 32, die die Welle 4 senkrecht zu deren Drehzentrum 6 durchragt und an der Welle 4 befestigt ist, verlaufen in Nuten 35,36. Die Länge der Nuten 35,36 sind so bemessen, daß die Drehbewegung der Welle 4 auf 7° begrenzt ist. Die Nuten 35,36, die in der FIG 2 gezeigt sind, verlaufen in Richtung der Zeichungsebene.

Im Rahmen der Erfindung wird vorgeschlagen, daß die Halterung drei Kassettenhalterungen in einem Winkelabstand von jeweils 120° trägt. Durch diesen Winkelabstand zwischen den Kassettenhalterungen sind die geparkten Kassettenhalterungen nicht im Wege, wenn die dritte Kassettenhalterung in eine Aufnahmelage gebracht worden ist.

Dadurch, daß die Halterung 8 an der horizontalen Welle 4 mit einem Winkel von 45° gegenüber dem Drehzentrum 6 der Welle 4 angebracht ist, ist erreicht worden, daß die Halterung 8 in ihrem Aufbau verhältnismäßig klein und einfach gemacht werden kann, gleichzeitig damit, daß die Kassettenhalterungen 9,10 in eleganter Weise von einer Aufnahmelage in eine Parklage gewechselt werden können.

## Patentansprüche

1. Röngendiagnostikgerät für Mammografieuntersuchungen mit einer Röntgenröhre (3), die über eine horizontale Welle (4) um ein in einem Stativ (1) angeordnetes Lager (5) drehbar ist, und mit mindestens zwei Kassettenhalterungen (9,10), die wahlweise in eine Aufnahmelage gebracht werden können, wobei die Kassettenhalterungen (9,10) um die horizontale Welle (4) derart schwenkbar sind, daß sich, wenn eine Kassettenhalterung (9 oder 10) in die Aufnahmelage gebracht ist, die andere Kassettenhalterung (10 oder 9) in einer Parklage befindet, **dadurch gekennzeichnet**, daß die Drehachse (6) der horizontalen Welle (4) etwa achsfluchtend mit der Mitte des Untersuchungsobjekts (7) angeordnet ist und daß die Kassettenhalterungen (9,10) in einer Halterung (8), die an der horizontalen Welle (4) drehbar angebracht ist, derart befestigt sind, daß sich, wenn die eine Kassettenhalterung (9,10) in die Aufnahmelage gebracht ist, die andere Kassettenhalterung (9,10) vom Untersuchungsobjekt (7) her gesehen, in einer Parklage hinter der Röntgenröhre (3) befindet, wobei die Drehung der Halterung (8) um die Welle (4) derart erfolgt, daß die Kassettenhalterungen (9,10) jeweils etwa denselben Abstand zur Drehachse (6) der horizontalen Welle (4) aufweisen, wenn sie sich in der Aufnahmelage befinden.

2. Röntgendiagnostikgerät nach Anspruch 1, **dadurch gekennzeichnet**, daß die Röntgenröhre (3) in einem Gehäuse (2) angebracht ist, das an der horizontalen Welle (4) befestigt ist.

3. Röntgendiagnostikgerät nach Anspruch 1 oder 2, **dadurch gekennzeichnet**, daß die Halterung (8) an der horizontalen Welle (4) in einem Winkel von etwa 45° angebracht ist.

4. Röntgendiagnostikgerät nach einem der Ansprüche 1 bis 3, **dadurch gekennzeichnet,** daß die Halterung (8), zwei Kassettenhalterungen (9,10), in einem Winkelabstand von 180° trägt.

5. Röntgendiagnostikgerät nach einem der Ansprüche 1 bis 3, **dadurch gekennzeichnet**, daß die Halterung (8) drei Kassettenhalterungen (9,10) in einem Winkelabstand von jeweils 120° trägt.

6. Röntgendiagnostikgerät nach einem der Ansprüche 1 bis 5, **dadurch gekennzeichnet**, daß an der horizontalen Welle (4) ein Lagergehäuse (12) angebracht ist, an dem eine Lagerscheibe (16) in einem Winkel von etwa 45° zur genannten Welle(4) befestigt ist, wobei die Halterung (8) an der Lagerscheibe (16) angebracht ist.

7. Röntgendiagnostikgerät nach Anspruch 6, **dadurch gekennzeichnet**, daß das Lagergehäuse (12) mit mindestens einem Flansch (15) versehen ist, an dem die Lagerscheibe (16) befestigt ist.

8. Röntgendiagnostikgerät nach Anspruch 6 oder 7, **dadurch gekennzeichnet**, daß die Halterung (8) mittels eines Ringes (19) und eines Deckels (20) für den Ring (19) um die Lagerscheibe (16) drehbar angebracht ist, wobei zwischen der Lagerscheibe (16) und dem Ring (19) und der Lagerscheibe (16) und dem Deckel (20), Gleitlager (21) angeordnet sind.

9. Röntgendiagnostikgerät nach einem der Ansprüche 6 bis 8, **dadurch gekennzeichnet**, daß der Deckel (20) oder der Ring (19) mit Arretiernuten (22,23) versehen ist, in die ein Verschlußteil (24) zum Arretieren der Kassettenhalterungen (9,10) in einer Aufnahmelage herabsenkbar ist.

10. Röntgendiagnostikgerät nach einem der Ansprüche 1 bis 9, **dadurch gekennzeichnet**, daß die horizontale Welle (4), die zusammen mit der Röntgenröhre (3), dem Lagergehäuse (12) und der Halterung (8) für die Kassettenhalterungen (9,10) um das Lager (5) im Stativ (1) drehbar ist, mittels eines Verschlußkragens (29) mit Arretierlagen (30) und mittels eines Verschlußteils (31), das mit dem Verschlußkragen (29) in einer der Arretierlagen (30) verschließbar ist, in bestimmten Lagen arretierbar ist.

11. Röntgendiagnostikgerät nach Anspruch 10, **dadurch gekennzeichnet**, daß der Verschlußkragen (29) die Welle (4) umschließt und am Stativ (1) befestigt ist, wobei das Verschlußteil (31) in Verbindung mit der Welle (4) angebracht ist.

## Claims

1. X-ray diagnosis apparatus for mammography, with an X-ray tube (3) which can be pivoted via a horizontal shaft (4) about a bearing (5) which is arranged in a stand (1), and with at least two cassette holders (9, 10) which can be brought alternately into a recording position, it being possible for the cassette holders (9, 10) to be swivelled about the horizontal shaft (4) in such a way that when one cassette holder (9 or 10) is brought into the recording position, the other cassette holder (10 or 9) is located in a standby position, characterized in that the axis of rotation (6) of the horizontal shaft (4) is arranged approximately in axial alignment with the centre of the examined object (7), and in that the cassette holders (9, 10) are secured in a mounting (8), arranged rotatably on the horizontal shaft (4), in such a way that when the one cassette holder (9, 10) is brought into the recording position, the other cassette holder (9, 10), viewed from the examined object (7), is located in a standby position behind the X-ray tube (3), the mounting (8) rotating about the shaft (4) in such a way that the cassette holders (9, 10) are in each case at approximately the same distance from the axis of rotation (6) of the horizontal shaft (4) when they are in the recording position.

2. X-ray diagnosis apparatus according to Claim 1, characterized in that the X-ray tube (3) is arranged in a housing (2) which is secured on the horizontal shaft (4).

3. X-ray diagnosis apparatus according to Claim 1 or 2, characterized in that the mounting (8) is arranged on the horizontal shaft (4) at an angle of approximately 45°.

4. X-ray diagnosis apparatus according to one of Claims 1 to 3, characterized in that the mounting (8) carries two cassette holders (9, 10) at an angle distance of 180°.

5. X-ray diagnosis apparatus according to one of Claims 1 to 3, characterized in that the mounting (8) carries three cassette holders (9, 10) at an angle distance of in each case 120°.

6. X-ray diagnosis apparatus according to one of Claims 1 to 5, characterized in that a bearing housing (12) is arranged on the horizontal shaft (4), on which bearing housing (12) a bearing disc (16) is secured at an angle of approximately 45° to the said shaft (4), the mounting (8) being arranged on the bearing disc (16).

7. X-ray diagnosis apparatus according to Claim 6, characterized in that the bearing housing (12) is provided with at least one flange (15) on which the bearing disc (16) is secured.

8. X-ray diagnosis apparatus according to Claim 6 or 7, characterized in that the mounting (8) is arranged rotatably about the bearing disc (16) by means of a ring (19) and a cover (20) for the ring (19), slide bearings (21) being arranged between the bearing disc (16) and the ring (19) and between the bearing disc (16) and the cover (20).

9. X-ray diagnosis apparatus according to one of Claims 6 to 8, characterized in that the cover (20) or the ring (19) is provided with locking nuts (22, 23) into which a closure part (24) can be lowered for locking the cassette holders (9, 10) in a recording position.

10. X-ray diagnosis apparatus according to one of Claims 1 to 9, characterized in that the horizontal shaft (4), which can be rotated about the bearing (5) in the stand (1) together with the X-ray tube (3), the bearing housing (12) and the mounting (8) for the cassette holders (9, 10), can be locked in defined positions by means of a closure collar (29) with locking positions (30) and by means of a closure part (31) which can be closed with the closure collar (29) in one of the locking positions (30).

11. X-ray diagnosis apparatus according to Claim 10, characterized in that the closure collar (29) surrounds the shaft (4) and is secured on the stand (1), the closure part (31) being arranged in connection with the shaft (4).

## Revendications

1. Appareil de radiodiagnostic pour des examens mammographiques, comportant un tube à rayons X (3), qui peut tourner à l'aide d'un arbre horizontal (4) autour d'un palier (5) disposé dans un statif (1), et comportant au moins deux supports (9,10) pour cassettes qui peuvent être amenés au choix dans une position de réception, les supports (9,10) pour cassettes pouvant basculer autour de l'arbre horizontal (4) de telle sorte que, lorsqu'un support (9c) pour cassette est amené dans la position de prise de vue, l'autre support (10 ou 9) pour cassette est situé dans une position d'attente, caractérisé par le fait que l'axe de rotation (6) de l'arbre horizontal (4) est disposé approximativement en alignement avec le centre de l'objet d'examen (7) et que les supports (9,10) pour cassettes sont fixés dans un support (8), qui est monté de manière à pouvoir tourner sur l'arbre horizontal (4) de telle sorte que, lorsqu'un support (9,10) pour cassette est amené dans la position de prise de vue, l'autre support (9,10) pour cassette est situé, lorsqu'on regarde à partir de l'objet d'examen (7), dans une position d'attente en arrière du tube à rayons X (3), la rotation du support (8) s'effectuant autour de l'arbre (4) de telle sorte que les supports (9,10) pour cassettes sont respectivement situés approximativement à la même distance de l'axe de rotation (6) de l'arbre horizontal (4) lorsqu'ils sont situés dans la position d'attente.

2. Appareil de radiodiagnostic suivant la revendication 1, caractérisé par le fait que le tube à rayons X (3) est logé dans un boîtier (2), qui est fixé à l'arbre horizontal (4).

3. Appareil de radiodiagnostic suivant la revendication 1 ou 2, caractérisé par le fait que le support (8) est monté sur l'arbre horizontal (4) sous un angle d'environ 45°.

4. Appareil de radiodiagnostic suivant l'une des revendications 1 à 3, caractérisé par le fait que le support (8) porte deux supports (9,10) pour cassettes, à une distance angulaire de 180°.

5. Appareil de radiodiagnostic suivant l'une des revendications 1 à 3, caractérisé par le fait que le support (8) porte trois supports (9,10) pour cassettes, à une distance angulaire de respectivement 120°.

6. Appareil de radiodiagnostic suivant l'une des revendications 1 à 5, caractérisé par le fait que sur l'arbre horizontal (4) est monté un logement de palier (12), auquel est fixé un disque de support (16) sous un angle d'environ 45° par rapport audit arbre (4), le support (8) étant monté sur le disque de support (16).

7. Appareil de radiodiagnostic suivant la revendication 6, caractérisé par le fait que le logement de palier (12) comporte au moins une bride (15), à laquelle est fixé le disque de support (16).

8. Appareil de radiodiagnostic suivant l'une des revendications 6 ou 7, caractérisé par le fait que le support (8) est disposé au moyen d'une bague (19) et d'un couvercle (20) pour la bague (19), de manière à pouvoir tourner autour du disque de palier (16), des paliers lisses (21) étant disposés entre la coque de palier (16) et la bague (19) et entre la coque de palier (16) et le couvercle (20).

9. Appareil de radiodiagnostic suivant l'une des revendications 6 à 8, caractérisé par le fait que le couvercle (20) ou la bague (19) est équipé de rainures de blocage (22,23), dans lesquelles un élément de fermeture (24) servant à bloquer les supports (9,10) pour cassettes peut être abaissé dans une position de prise de vue.

10. Appareil de radiodiagnostic suivant l'une des revendications 1 à 9, caractérisé par le fait que l'arbre horizontal (4), qui peut tourner autour du palier (5) dans le statif (1) conjointement avec le tube à rayons X (3), le logement de palier (12) et le support (8) pour les supports (9,10) pour cassettes, peut être arrêté dans des positions déterminées à l'aide d'un collet de fermeture (29) comportant des positions de fermeture (30), et à l'aide d'un élément de fermeture (31), qui peut être fermé au moyen du collet de fermeture (29) dans l'une des positions d'arrêt (30).

11. Appareil de radiodiagnostic suivant la revendication 10, caractérisé par le fait que le collet de fermeture (29) entoure l'arbre (4) et est fixé sur le statif (1), l'élément de fermeture (31) étant monté en liaison avec l'arbre (4).
